# EUROPEAN PATENT SPECIFICATION

(11) **EP 3 993 822 B1**
(45) Date of publication and mention of the grant of the patent: **07.06.2023**
(21) Application number: 20736956.2
(22) Date of filing: 01.07.2020
(51) Int. Cl.: A61K 38/17, A61P 25/16, A61P 25/28, A61K 9/00

(54) **AMYLOID BETA PEPTIDE FOR NASAL TREATMENT OF TAU PROTEIN-RELATED DISORDERS**
AMYLOID BETA PEPTID ZUR NASALE BEHANDLUNG VON TAU-PROTEIN-ABHÄNGIGEN ERKRANKUNGEN
PEPTIDE AMYLOIDE BETA POUR LE TRAITEMENT NASAL DE TROUBLES LIÉS À LA PROTÉINE TAU

(30) Priority: 02.07.2019 IT 201900010722
(43) Date of publication of application: 11.05.2022
(60) Divisional application: 23151657.6
(73) Proprietor: Fondazione IRCCS Istituto Neurologico Carlo Besta, 20133 Milano (IT)
(72) Inventor: TAGLIAVINI, Fabrizio, 20133 Milano (IT); DI FEDE, Giuseppe, 20133 Milano (IT); SALMONA, Mario, 20156 Milano (IT)
(74) Representative: Gerli, Paolo
(86) International application number: PCT/EP2020/068466
(87) International publication number: WO 2021/001405

(56) References cited:
- WO-A2-2009/047002
- GIUSEPPE DI FEDE ET AL: "Translational Research in Alzheimer's and Prion Diseases", JOURNAL OF ALZHEIMER'S DISEASE, vol. 62, no. 3, 13 March 2018 (2018-03-13) , pages 1247-1259, XP055676084, NL ISSN: 1387-2877, DOI: 10.3233/JAD-170770
- LUISA DIOMEDE ET AL: "The new [beta] amyloid-derived peptide A[beta]1-6A2V-TAT(D) prevents A[beta] oligomer formation and protects transgenic C. elegans from A[beta] toxicity", NEUROBIOLOGY OF DISEASE, vol. 88, 1 April 2016 (2016-04-01), pages 75-84, XP055675954, AMSTERDAM, NL ISSN: 0969-9961, DOI: 10.1016/j.nbd.2016.01.006
- GIUSEPPE DI FEDE ET AL: "Tackling amyloidogenesis in Alzheimer's disease with A2V variants of Amyloid-[beta]", SCIENTIFIC REPORTS, vol. 6, no. 1, 11 February 2016 (2016-02-11), XP055675953, DOI: 10.1038/srep20949

## Description

### FIELD OF THE INVENTION

The present invention belongs to the field of the treatment and prevention of neurodegenerative diseases. A new therapeutic approach is herein presented, targeted to prevent the pathological activity of misfolded tau protein in patients in need thereof.

### STATE OF THE ART

In consequence of the prolonged life expectancy in modern society, neurodegenerative diseases and the related cognitive impairment are becoming more and more present among the elderly population. Alzheimer's Disease (AD) represents the most frequent form of dementia in the elderly, afflicting an estimated 6 million people in the EU and over 30 million individuals worldwide. These figures are projected to increase substantially as the world population rapidly ages. The impact on the quality of life of patients and their families is severe, accompanied by immense psychological pain; furthermore, the massive economic burden associated with AD must be considered. For those compelling reasons, the identification of effective therapeutic strategies is considered a priority by worldwide health organizations, both public and private, and acted upon accordingly.

In AD patients, the misfolded amyloid β (Aβ) peptides accumulate in the form of amyloid oligomers, amyloid fibrils and amyloid plaques in the brain tissue¹ with associated neurotoxic effects believed responsible for the onset and progression of AD. In 2009, based on a clinical case, the present inventors described a genetic variant of Aβ with the exciting ability to hinder amyloidogenesis². This mutation, also described by the present inventors in the patent publication EP 2 220 251, consisted of an Alanine-to-Valine substitution (Aβ_{A2V} or A2V) in position 2 of the Aβ sequence, corresponding to the 673 codon in the Amyloid Precursor Protein (*APP*) gene (*APP*_{A673V}). This mutation was found in the homozygous state in a patient with an early-onset AD and in his younger sister who developed the disease; but, notably, heterozygous carriers of the same family were not affected even in advanced age. These data were consistent with an autosomal recessive pattern of inheritance of the A673V genetic defect in this family - at odds with all the other previously reported dominant mutations associated with AD. The performing cognitive capacity of the eldest A673V heterozygous carriers in this family prompted the present inventors to hypothesize that this mutation has protective effects in the heterozygous state².

More recently, another variant, involving the same codon, was found to be protective against AD. The authors showed, based on the whole-genome sequence data from 1,795 Icelanders, that those elderly people had natural protection against AD in the presence of an Aβ variant characterized by an Alanine-to-Threonine substitution in position 2 (*APP*_{A673T}, Aβ_{A2T} or A2T variant).

Further studies were carried out *in vitro* with a prototypic compound consisting of an all-D-isomer synthetic peptide limited to the first six amino acids of the N-terminal sequence of the A2V-mutated Aβ, namely, Aβ1-6_{A2V}(D), which displayed an extraordinary ability to interact with full-length wild-type (WT) Aβ, interfering with its nucleation or nucleation-dependent polymerization 2,4. Moreover, molecular dynamics simulations showed that the anti-amyloidogenic activity of the mutated Aβ1-6_{A2V}(D) short-peptide is related to its structural flexibility (open-to-closed conformational flexibility), in contrast to the structural rigidity of the analogue wild-type sequence (closed conformation). This short-peptide is yet unable to cross the blood-brain barrier. In previous attempts to ease brain permeation, the peptide was linked to the segment of HIV-related TAT protein: unfortunately, the desired increased brain penetration was accompanied by an undesired increase of brain amyloid burden - due to the intrinsic pro-amyloidogenic abilities of the carrier (REF: Giunta, B. et al. HIV-1 "Tat contributes to Alzheimer' s disease-like pathology in PSAPP mice". Int J Clin Exp Pathol 2, 433-443 (2009); Kim, J., Yoon, J. H. & Kim, Y. S. HIV-1 "Tat interacts with and regulates the localization and processing of amyloid precursor protein". PLoS One 8, e77972, doi: 10.1371/journal.pone.0077972 (2013) - which potentially opposes to the therapeutic effect of the Aβ1-6_{A2V}(D) peptide and increases the risk of amyloid plaque formation⁵; this highlights a difficulty to concentrate the therapeutic effectiveness of this peptide in the brain, i.e. where it is most needed.

Until today, neurodegenerative disorders remain exceedingly complex, and biochemical strategies proposed for their treatment have often proved unsatisfactory: for example, in case of AD, more than 450 clinical trials seeking AD-modifying drugs to impinge on the pathway of Aβ aggregation and plaque formation have failed dramatically, and there are still no treatments. The reasons for the consistent failure of pharmacological trials for AD are unclear and surely not simple to be elucidated⁶. Different factors may have contributed⁷, and one hypothesis is that the tested drugs aimed at specific single targets, without taking into account the complexity of the disorder. Another probable limitation is that the putative AD-modifying drugs were mainly designed for targeting candidates *in vitro⁸,* and on evidence deriving primarily from preclinical observations in AD animal models⁹; these experimental designs and models might not sufficiently account for the real-world complexity of the actual disease.

Similarly, no effective treatments are available for other tauopathies (i.e., neurodegenerative diseases due to abnormal accumulation of tau in the central nervous system), and the development of a successful strategy for brain delivery of therapeutics to treat tauopathies is still a big challenge (REF: Panza F, Lozupone M, Seripa D, Daniele A, Watling M, Giannelli G, Imbimbo BP. "Development of disease-modifying drugs for frontotemporal dementia spectrum disorders". Nat Rev Neurol. 2020 Apr;16(4):213-228. doi: 10.1038/s41582-020-0330-x; Hanes J, Dobakova E, Majerova P. "Brain Drug Delivery: Overcoming the Blood-brain Barrier to Treat Tauopathies". Curr Pharm Des. 2020;26(13):1448-1465. doi: 10.2174/1381612826666200316130128). At the moment, only symptomatic drugs are used in the common clinical practice to cure the patients affected by neurodegenerative disorders related to tau pathology, even if some promising therapeutic strategies - based on a single-target approach - wait for validation in clinical trials (REF: VandeVrede L, Boxer AL, Polydoro M."Targeting tau: Clinical trials and novel therapeutic approaches". Neurosci Lett. 2020 Jul 13;731:134919. doi: 10.1016/j.neulet.2020.134919).

In view of the above, there is still an unmet need for improved therapies against neurodegenerative diseases caused by tau, in particular being capable of concentrating neuroprotective effects at the level of central nervous system; the need is also felt for therapeutic strategies capable to simultaneously inhibit a multiplicity of neurotoxic events, thus enhancing the effectiveness of the treatment vis à vis the complex, variable and multi-factor nature of neurodegenerative disorders.

### SUMMARY

Tau protein misfolding, aggregation and accumulation is a key event in several neurological disorders, leading to synaptic loss and neurodegeneration¹⁰. In Alzheimer's disease tau pathology is associated with misfolding of Aβ, -while in primary tauopathies it occurs spontaneously or as a consequence of *MAPT* mutations. The present inventors have found that the hexapeptide Aβ1-6_{A2V}(D) is capable of interfering with tau-related abnormal processes at multiple levels, in particular by inhibiting the binding of Aβ1-42 oligomers to tau and hindering the polymerization of full-length tau. These actions are associated with additional, newly identified neuroprotective effects of Aβ1-6_{A2V}(D), namely the inhibition of oxygen radicals production induced by Aβ peptides and the prevention of synaptic dysfunction. The combination of these varied activities in a single drug makes available an ideal therapeutic asset for the treatment of diseases related to tau pathology. The object of the invention is, therefore, the provision of the hexapeptide Aβ1-6_{A2V}(D) for use in a multi-target method of treating or preventing diseases related to tau pathology, said diseases being typically neurodegenerative. The invention represents the first example of a multi-target disease-modifying approach to tau protein related disorders, enabling a more effective treatment or prevention of the related diseases. In a preferred embodiment, Aβ1-6_{A2V}(D) is formulated for/administered by the intranasal route, obtaining an unexpected, impressive level of diffusion of the peptide through the blood-brain barrier, with effective delivery of the peptide in the most important brain areas.

### DESCRIPTION OF THE FIGURES

**Figure 1****.** Time-course of Aβ1-42 aggregation. Aβ1-6A2V(D) binds selectively to Aβ oligomers as determined by thioflavin T dye (ThT) fluorimetric assay and hinders Aβ1-42 aggregation. Aβ1-42 oligomers were prepared according to Beeg et al¹¹.
**Figure 2****.** Human β-Secretase Inhibition by Aβ1-6_{A2V}(D).
**Figure 3****.** Mouse brain distribution of Aβ1-6_{A2V}(D) peptide after intranasal administration. Mice were sacrificed 4 (A), 24 (B) and 48 (C) hours after the last administration.
**Figure 4****.** Mouse brain distribution of Aβ1-6_{A2V} (D) peptide 2 hours after the last intranasal administration: A) Control and B) Treated Mice.
**Figure 5****.** Effect of Aβ1-6_{A2V}(D) on Aβ oligomer formation in the brain: biochemical assessment. APPSwe/PS1DE9 were treated with Aβ1-6_{A2V}(D) at 50mg/kg for five months. Control group was treated intranasally with PBS. A: Levels of Aβ aggregates in soluble and insoluble brain fractions after 2,5 months of treatment. B: Levels of Aβ aggregates in soluble and insoluble brain fractions after five months of treatment. * p<0,01; *** p<0,001.
**Figure 6****.** Effect of Aβ1-6_{A2V}(D) on Aβ deposits in mouse brain: neuropathological assessment. A: APPSwe/PS1DE9 were treated with Aβ1-6_{A2V}(D) daily for five months at 50mg/kg. Control group (CTRL) was treated intranasally with PBS. Amyloid burden measured by plaque count. ** p<0,01; *** p<0,001. B: Immunohistochemistry with the 4G8 anti-Aβ antibody.
**Figure 7****.** Effect of Aβ1-6_{A2V}(D) on synaptopathy. Subcellular fractionation of hippocampal homogenates from mice treated with Aβ1-6_{A2V(}D) (right columns) or PBS (left columns). Western blot analysis with antibodies against synaptic receptors (NMDA-2A, GluN2B, GluA1 and GluA2) and scaffold protein (PSD95). The treatment with Aβ1-6_{A2V}(D) preserved synaptic integrity.
**Figure 8****.** Binding of different Aβ1-42 assemblies to Immobilized Tau
**Figure 9****.** Aβ1-6_{A2V}(D) inhibits the binding Aβ1-42 oligomers to Tau. The IC50 obtained in two independent experiments were 360.2 and 387.6 µM.
**Figure 10****.** Tau aggregation was triggered by heparin in the tau/EPA ratio of 4:1 (w/w) in the presence of 5mM dithiothreitol (DTT).
**Figure 11****.** Time course of WT full-length tau aggregation in the absence and presence of Aβ1-6_{A2V}(D) as determined by atomic force microscopy (AFM). Tau alone forms fibres whose numbers and size increase with time of incubation. Following the co-incubation with Aβ1-6_{A2V}(D), the number of fibres is significantly reduced, and an increase of granular structure was evident. Color scale: 0-150mV.
**Figure 12****.** Time course of full-length tau P301L aggregation in the absence and presence of Aβ1-6_{A2V}(D) as determined by AFM. Tau P301L alone shows an increased fibrils formation with time. In the presence of Aβ1-6A2V(D) the number of fibres is significantly reduced, and an increase of granular structure was evident. Color scale: 0-150mV.

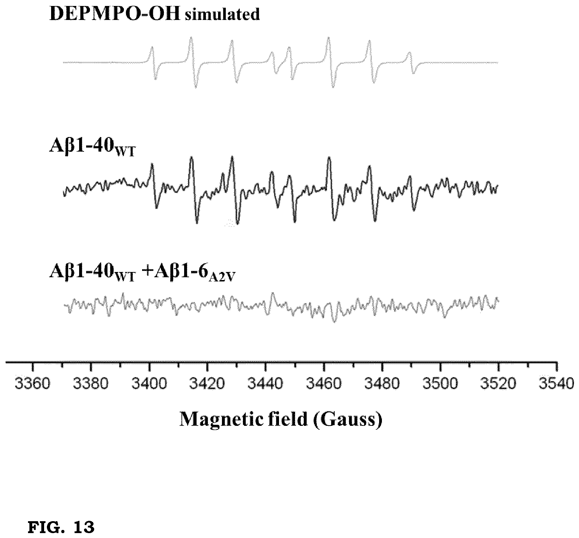

**Figure 13****.** The DEPMO-OH-spin trap assay for the detection of ROS formation during the Cu(II)- or Fe(III)-Aβ-catalyzed processes. Aβ1-40_{WT} spontaneously produces OH radicals in PBS buffer containing traces of Cu (II) and Fe (III). When co-incubated with Aβ1-6A2V(D) the production of OH radicals is significantly reduced.

### DETAILED DESCRIPTION OF THE INVENTION

The peptide Aβ1-6_{A2V}(D) used in the present invention is known as such ^{2, 4}: it is the all-D-isomer synthetic peptide corresponding to the first six amino acids of the N-terminal sequence of the A2V-mutated Aβ; its amino acid sequence, DVEFRH, and its method of synthesis are disclosed by EP 2 220 251; all these publications are incorporated herein by reference.

The object of the present invention is the hexapeptide Aβ1-6_{A2V}(D) for use in a multi-targeted method of treating diseases related to tau protein activity. A further object is the use of hexapeptide Aβ1-6_{A2V}(D) in the manufacturing of a medicament for a multi-targeted method of treating diseases related to tau protein activity. A further object of the invention is a method of multi-target treating diseases related to tau protein activity, comprising administering the hexapeptide Aβ1-6_{A2V}(D) to a patient in need thereof.

The terms "multi-targeted treatment" or "multi-target treatment" mean herein the effectiveness of Aβ1-6_{A2V}(D) to interfere simultaneously at different levels with the process of tau protein misfolding and accumulation; such targets, i.e. effects of Aβ1-6_{A2V}(D), include: inhibiting the binding Aβ1-42 oligomers to tau protein, , hindering polymerization of full-length tau protein and inhibiting β-secretase activity; these effects are advantageously associated with other neuroprotective effects of Aβ1-6_{A2V}(D), also found herein for the first time, namely: inhibiting oxygen radicals production by β-amyloid peptides and inhibiting synaptic dysfunction. The combination of these effects in a single drug makes available a potent therapeutic asset for the treatment of diseases related to tau pathology.

There is no limitation to the specific diseases related to tau protein which can be an object of the present treatment. These are typical of neurodegenerative type; among them, the following can be mentioned: Ageing-related tau astrogliopathy, Alzheimer's Disease, Argyrophilic grain disease, Chronic traumatic encephalopathy, Corticobasal degeneration, Diffuse neurofilament tangles with calcification, Frontotemporal dementia, Globular glial tauopathies, Parkinsonism linked to chromosome 17, Pick disease, Postencephalitic parkinsonism, Primary age-related tauopathy, Progressive supranuclear palsy. The present invention enables to treat with the same drug, Aβ1-6_{A2V}(D), the group of tau protein-related diseases which either had no prior treatment or were addressed separately, based on their specific symptoms to be cured.

The term "treatment" used herein includes any way of administering the hexapeptide Aβ1-6_{A2V}(D) to a patient in need thereof to obtain a therapeutic effect on a tau protein-related disease. The disease needs not necessarily to be ongoing at the time of administration of Aβ1-6_{A2V}(D), i.e. the patient to be treated may either be affected or be at risk of being affected by one or more of said diseases, obtaining respectively a curative or a preventive effect on such diseases.

To practice the present treatment, the hexapeptide Aβ1-6_{A2V}(D) may be administered by any known administration routes, e.g. orally, personally, buccally, intravenously, intrathecally, intramuscularly, topically, transdermally, inhalatorily, intranasally, intraocularly, etc. A particularly effective administration route, representing a preferred embodiment of the present invention, is the intranasal one: in fact, as reported in the experimental sections, the intranasal administration of hexapeptide Aβ1-6_{A2V}(D) had obtained very high levels of brain penetration of this peptide: this represents a key unexpected advantage, since this peptide does not permeate through the blood-brain-barrier when administered outside the brain; this effect is particularly important considering that tau protein-related diseases arise mainly within the central nervous system; further the brain penetration is herein obtained without need of using special carriers designed to enable the blood-brain-barrier passing: avoiding the use of these carriers results in lower production costs, positively impacting on the final drug product, also offering a better therapeutic profile by avoiding any unnecessary accumulation of carrier moieties in the brain. All pharmaceutical forms allowing intranasal delivery are usable in this embodiment: reference can be made to, e.g. nasal drops, nasal ointments, nasal inserts, nasal sprays, aerosols, inhalable powders, etc.

In line with the above, the term "pharmaceutical composition in form suitable for intranasal administration", refers herein and is limited to a commercial pharmaceutical product (i.e. packaged medicament) which is both formulated and explicitly presented to the user for the administration via the intranasal route: the formulation involves the choice of active and non-active ingredients being qualitativelty and quantitatively chosen to be compatible with contact with the intranasal mucosa, while the explicit presentation to the user is evident in the product as such by means of directions to administer the composition intranasally.

The present uses and treatments may be performed within a wide range of dosages of Aβ1-6_{A2V}(D), correlated to the disease type and stage and patient's age and conditions. Dosages are conveniently chosen by the skilled clinician taking the about factors into account; non-limitative daily dosages ranging from 0.1 and 100 mg/Kg. Advantageously when performed intranasally, the present treatment with Aβ1-6_{A2V}(D) does not require contiguous daily administrations: in the present experiments, effective concentrations of this peptide were found in the brain even 48 hours after administration.

Pharmaceutical dosage units allowing the administration of said dosages, via one or more administrations during the day, can be produced by standard pharmaceutical techniques; reference, non-limitative amounts of hexapeptide Aβ1-6_{A2V}(D) in said dose units ranges between 1 and 7,000 mg. Depending on the chosen administration route, the hexapeptide Aβ1-6_{A2V}(D) can be formulated in all possible pharmaceutical forms, for examples tablets, pills, capsules, microcapsules, dragees, lozenges, powders, granulates, microgranules, pellets, solutions, suspensions, emulsions, infusions, drops, aerosols, liposomes, creams, ointments, gels, bioadhesive films, bioadhesive patches, enemas, suppositories, etc.

The invention is now disclosed using the following non-limitative examples.

### EXPERIMENTAL SECTION

The present experiments were aimed at providing new insights into the biochemical effects of Aβ1-6_{A2V}(D), looking for unknown biochemical effects and exploring ways of enhancing the bioavailability of Aβ1-6_{A2V}(D) in the body districts affected by tau protein-related diseases, with particular reference to the central nervous system. The following results were found.

### A. Aβ1-6_{A2V}(D) selectively binds Aβ oligomers

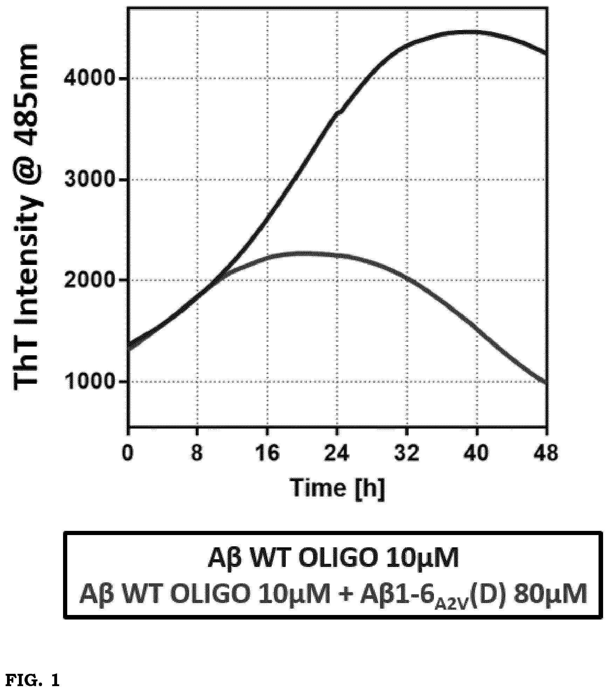

We have clarified that Aβ1-6_{A2V}(D) selectively binds to oligomers, as reported in Figure 1. This feature underlines the presence of a peculiar and selective mechanism of action that hinders the neurotoxicity of Aβ oligomers.

### B. Human β-Secretase (BACE1) Inhibition.

The major component of the amyloid plaques is aggregated Aβ which derived from sequential proteolytic cleavage of the amyloid precursor protein (APP), a transmembrane protein, by the action of β- and γ-secretases. The β-secretase cleavage is the first step in the generation of Aβ peptides, and the β-secretase 1 or β-amyloid-converting enzyme 1 is the major β-secretase; misfolding of the generated Aβ peptides leads to tau protein aggregation accompanied by synaptic loss and neurodegeneration.

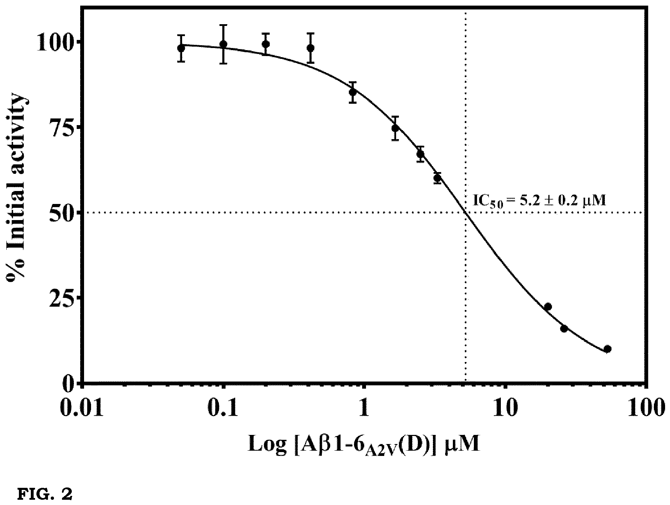

Figure 2 shows that Aβ1-6_{A2V}(D) inhibits in a dose-dependent manner the activity of the human recombinant β-secretase with an IC₅₀ value of 5.2±0.2 µM.

### C. In vivo effectiveness of intranasally administered Aβ1-6_{A2V}(D)

We studied if intranasal delivery of Aβ 1-6_{A2V}(D), not conjugated with any carrier, could provide therapeutically effective levels of this peptide in the brain. Aβ1-6_{A2V}(D) was dissolved in saline at the concentration of 125 mg/ml, and each mouse received 12 µl of this solution.

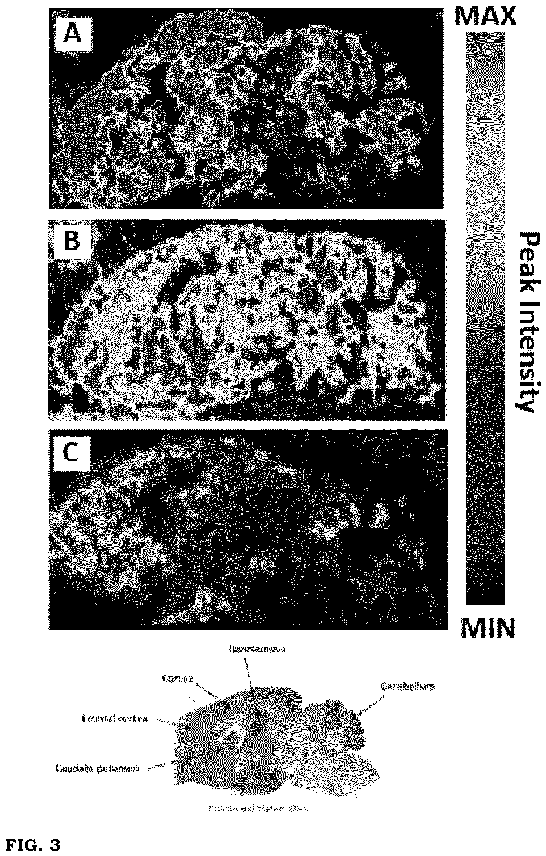

To this end, preliminarily wild-type mice were treated once a week for 4 weeks at a dose of 16.7 mg/kg. Animals were sacrificed 4, 24, 48 hours from the last intranasal delivery. We observed a consistent diffusion of the peptide in brain tissue involving areas critically implicated in amyloid deposition - such as the hippocampus - as revealed by MALDI-TOF imaging mass spectrometry (Figure 3). High levels of the peptide are present in the main brain areas (cerebral cortex, hippocampus, caudate putamen and cerebellum) after intranasal administration and as shown in the same figure, the peptide can be still observed in the cerebral cortex 48 hours after the last treatment.

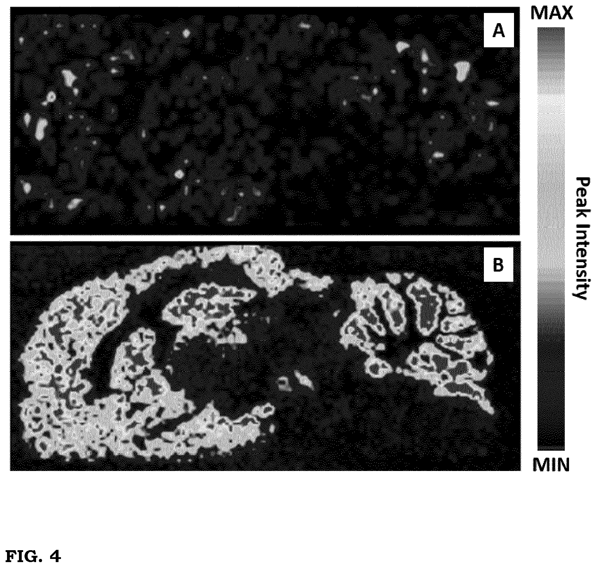

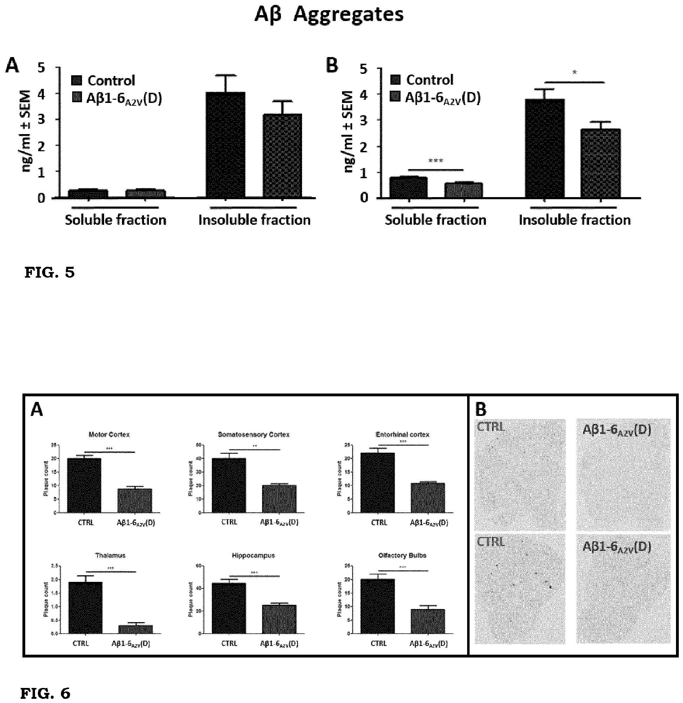

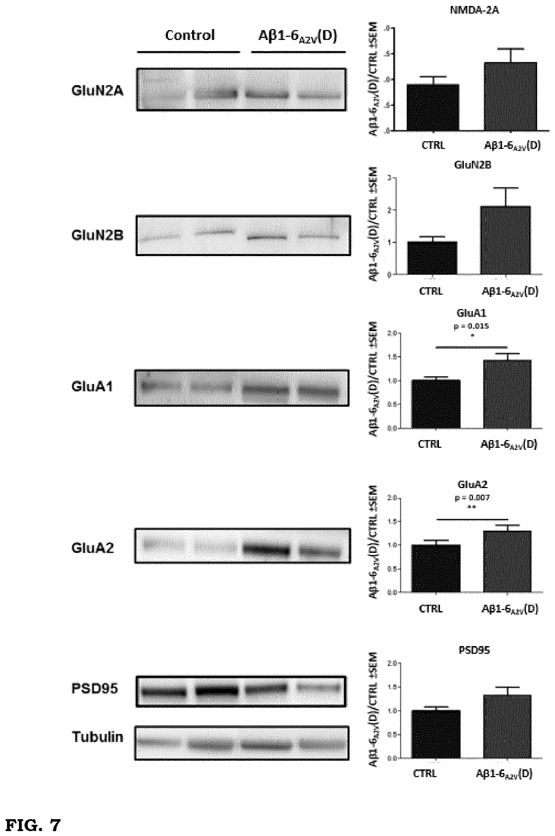

This observation prompted us to carry out a more comprehensive treatment schedule comprising MALDI-TOF imaging experiments for the determination of Aβ1-6_{A2V}(D) levels in the brain following intranasal administration for 5 months at 50 mg/kg every 48 hours. Following this approach, we treated the same transgenic mice used in the previous *in vivo* studies and obtained consistent anti-amyloidogenic effects even in the long-term treatment (5 months). In particular, the intranasal treatment with Aβ1-6_{A2V}(D) - without use of any blood-brain-barrier passing carrier, resulted in:
- consistent diffusion of the peptide in brain tissue involving areas critically implicated in amyloid deposition occurring in AD - such as the hippocampus - as revealed by MALDI-TOF imaging mass spectrometry (Figure 4);
- inhibition of oligomer generation and amyloid accumulation (Figures 5 and 6);
- prevention of synaptic dysfunction (Figure 7) even in the long-term treatment schedule.

In conclusion, the intranasal treatment with Aβ1-6_{A2V}(D) - without using any brain-targeting carriers - resulted in:
- inhibition of oligomer generation and amyloid accumulation;
- prevention of synaptic dysfunction even in the long-term treatment schedule.

Most importantly, very recent studies in our laboratories provided evidence in favour of the ability of the Aβ1-6_{A2V}(D) peptide of hindering the tau-related pathology in AD by two main molecular mechanisms:
1. Inhibition of the binding of Aβ oligomers to full-length wt tau
2. Inhibition of the aggregation of tau protein.

### D. Aβ1-6_{A2V}(D) inhibits Aβ1-42 oligomers binding to tau protein

The ability of Aβ oligomers to bind tau monomers may be a source of toxicity, e.g. by decreasing the population of tau needed to regulate microtubule dynamics. We used Surface Plasmon Resonance (SPR) to investigate whether or not Aβ1-6_{A2V}(D) can inhibit the binding of Aβ1-42 oligomers to tau (2N4R-Tau) monomers. First, we found that tau binds Aβ1-42 oligomers but not Aβ1-42 monomers or fibrils.

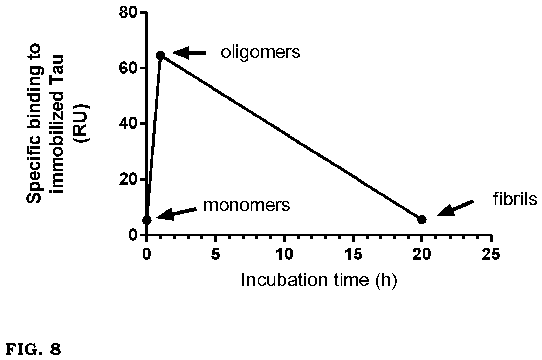

Figure 8 shows the binding behaviour of different assemblies formed during aggregation of 100 µM Aβ1-42 in PBS to tau immobilized on the SPR chip surface. The negligible binding signal was observed injecting the solution at t=0 (monomers) or incubated for 24h (fibrils). The significant binding was found injecting the solution preincubated for one hour. We previously demonstrated that this solution is enriched with small, toxic oligomers^{12, 13}.

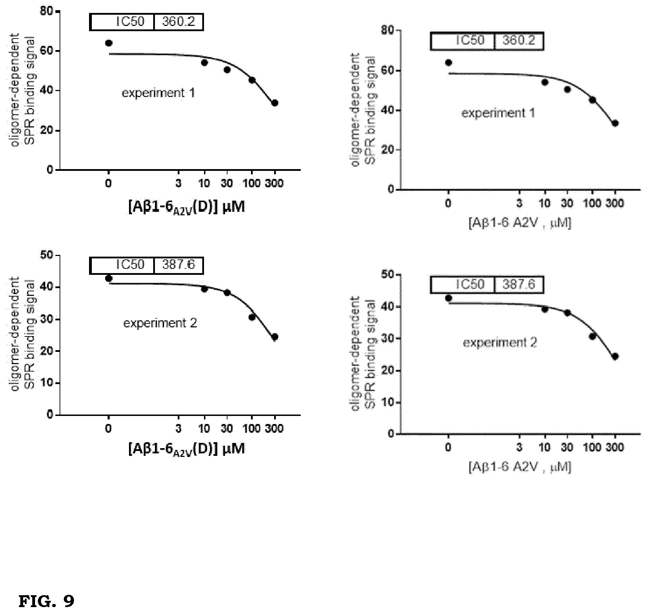

Then we demonstrated that Aβ1-6_{A2V}(D) inhibits Aβ1-42 oligomers binding to tau. For this, oligomers were diluted into PBST containing various concentrations of Aβ1-6_{A2V}(D) and equilibrated for one hour at room temperature, before injecting into the SPR apparatus. The oligomer-dependent SPR binding signal was inhibited by Aβ1-6_{A2V}(D) in a concentration-dependent manner (Figure 9).

### E. Aβ1-6_{A2V}(D) hinders the polymerization of full-length tau

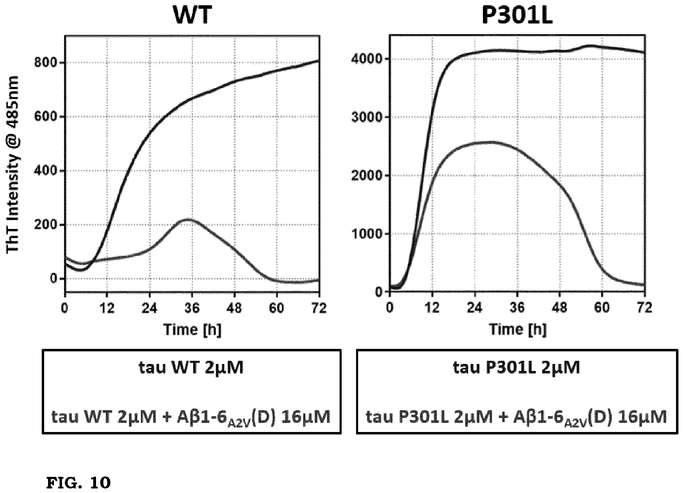

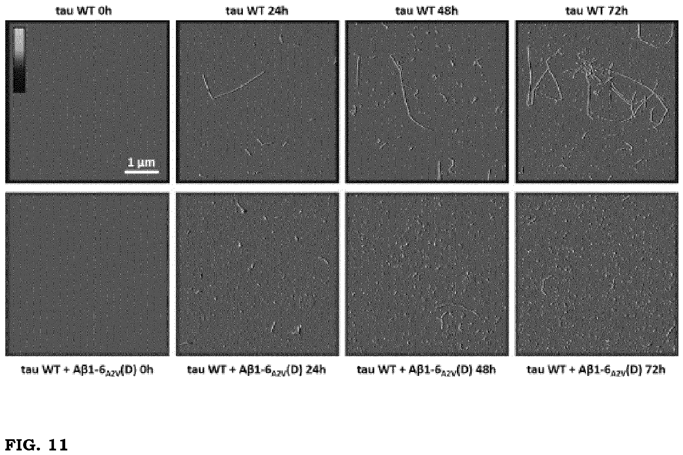

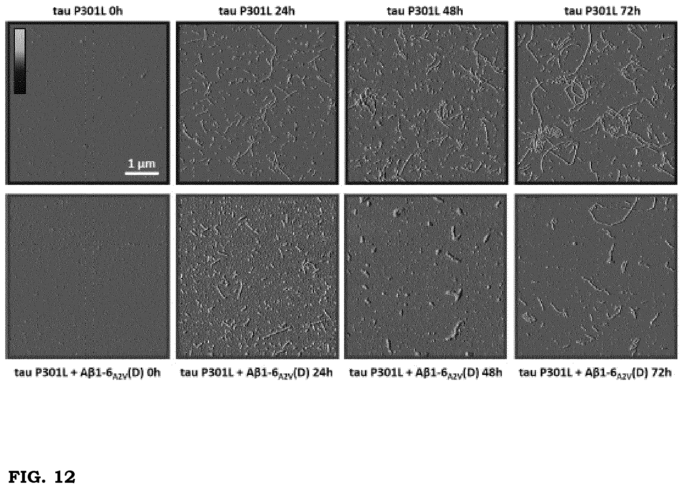

This peptide, when incubated with wt full-length tau or P301L tau, significantly **reduces the capacity of these proteins to polymerize** as deduced by the ThT test (Figure 10). This was also confirmed by atomic force microscopy analysis, as reported in Figures 11 and 12.

### F. Aβ1-6_{A2V}(D) hinders the reactive oxygen generation by Aβ-catalyzed redox processes.

This peptide, when incubated with Aβ1-40WT, inhibits the spontaneous formation of OH radicals in a buffer containing traces of Cu (II) and Fe (III)

These data confirm the "multitargeting nature" of the present Aβ1-6_{A2V}(D) based strategy and open the way to the therapeutic use of this approach for the treatment and/or prevention of neurodegenerative tauopathies.

### References

1. Murphy MP, LeVine H, 3rd. Alzheimer's disease and the amyloid-beta peptide. J Alzheimers Dis 2010; 19(1): 311-323.
2. Di Fede G. A recessive mutation in the APP gene with dominant-negative effect on amyloidogenesis (vol 323, pg 1473, 2009). Science 2009; 325(5937): 147.
3. Jonsson T, Atwal JK, Steinberg S, Snaedal J, Jonsson PV, Bjornsson S et al. A mutation in APP protects against Alzheimer's disease and age-related cognitive decline. Nature 2012; 488(7409): 96-99.
4. Di Fede G, Catania M, Morbin M, Giaccone G, Moro ML, Ghidoni R et al. Good gene, bad gene: new APP variant may be both. Prog Neurobiol 2012; 99(3): 281-292.
5. Di Fede G, Catania M, Maderna E, Morbin M, Moda F, Colombo L et al. Tackling amyloidogenesis in Alzheimer's disease with A2V variants of Amyloid-beta. Scientific Reports 2016; 6**.**
6. Gayvert KM, Madhukar NS, Elemento O. A Data-Driven Approach to Predicting Successes and Failures of Clinical Trials. Cell Chem Biol 2016; 23(10): 1294-1301.
7. Mehta D, Jackson R, Paul G, Shi J, Sabbagh M. Why do trials for Alzheimer's disease drugs keep failing? A discontinued drug perspective for 2010-2015. Expert Opin Investig Drugs 2017; 26(6): 735-739.
8. Hilbich C, Kisters-Woike B, Reed J, Masters CL, Beyreuther K. Substitutions of hydrophobic amino acids reduce the amyloidogenicity of Alzheimer's disease beta A4 peptides. J Mol Biol 1992; 228(2): 460-473.
9. Franco R, Cedazo-Minguez A. Successful therapies for Alzheimer's disease: why so many in animal models and none in humans? Front Pharmacol 2014; 5: 146.
10. Morris GP, Clark IA, Vissel B. Inconsistencies and controversies surrounding the amyloid hypothesis of Alzheimer's disease. Acta Neuropathol Commun 2014; 2: 135.
11. Beeg M, Stravalaci M, Bastone A, Salmona M, Gobbi M. A modified protocol to prepare seed-free starting solutions of amyloid-β (Aβ)1-40 and Aβ1-42 from the corresponding depsipeptides. Anal Biochem. 2011; 411(2): 297-299.
12. Stravalaci M, Bastone A, Beeg M, Cagnotto A, Colombo L, Di Fede G et al. Specific Recognition of Biologically Active Amyloid-beta Oligomers by a New Surface Plasmon Resonance-based Immunoassay and an in Vivo Assay in Caenorhabditis elegans. Journal of Biological Chemistry 2012; 287(33): 27796-27805.
13. Stravalaci M, De Blasio D, Orsini F, Perego C, Palmioli A, Goti G et al. A New Surface Plasmon Resonance Assay for In Vitro Screening of Mannose-Binding Lectin Inhibitors. J Biomol Screen 2016; 21(7): 749-757.

## Claims

1. Peptide Aβ1-6_{A2V}(D) for use in a multi-target method of treating tau protein-related diseases, wherein said peptide is administered intranasally.

2. Peptide for the use according to claim 1, wherein the multi-target method is effective in inhibiting each of the following: binding of Aβ 1-42 oligomers to tau protein, polymerization of full-length tau protein; β-secretase activity.

3. Peptide for the use according to claims 1-2, wherein the treatment also inhibits oxygen radicals production induced by β-amyloid peptides and synaptic dysfunction.

4. Peptide for the use according to claims 1-3, wherein said tau protein-related diseases are one or more among: Aging-related tau astrogliopathy, Alzheimer's Disease, Argyrophilic grain disease, Chronic traumatic encephalopathy, Corticobasal degeneration, Diffuse neurofilament tangles with calcification, Frontotemporal dementia, Globular glial tauopathies, Parkinsonism linked to chromosome 17, Pick disease, Postencephalitic parkinsonism, Primary age-related tauopathy, Progressive supranuclear palsy.

5. Peptide for the use according to claim 1-4, wherein said peptide is administered as a unit dose comprised between 1 and 7,000 mg.

6. Peptide for the use according to claims 1-5, wherein said peptide is administered at daily dose ranging from 0.1 to 100 mg/kg.

7. Peptide for the use according to claims 1-6, wherein said peptide is formulated as a solution or spray or aerosol, for intranasal administration.

8. Peptide for the use according to claims 1-7, wherein said intranasally administered peptide accumulates in brain areas including cortex, hippocampus, caudate putamen, cerebellum.

9. Peptide for the use according to claims 1-8, wherein said treatment does not require daily administrations, being effective for up 48 hours after administration.

10. Pharmaceutical composition comprising the peptide Aβ1-6_{A2V}(D) in form suitable for intranasal administration.

11. Pharmaceutical composition according to claim 10, in the form of intranasal instillable solution, intranasal spray, intranasal powder, intranasal aerosol.

12. Pharmaceutical composition according to claims 10-11, wherein said peptide Aβ1-6_{A2V}(D) is present at a concentration ranging from 10 to 100 mg/ml.

13. Pharmaceutical composition according to claims 10-12, in form of a unit dose containing from 1 to 7,000 mg of peptide Aβ1-6_{A2V}(D).

## Patentansprüche

1. Aβ1-6_{A2V}(D) Peptid zur Verwendung in einem Multi-Target-Verfahren zur Behandlung von Tau-Protein-abhängigen Erkrankungen, wobei das Peptid intranasal verabreicht wird.

2. Peptid zur Verwendung gemäß Anspruch 1, wobei das Multi-Target-Verfahren bei der Hemmung jedes der Folgenden wirksam ist: Bindung von Aß1-42-Oligomeren an das Tau-Protein, Polymerisation des Tau-Proteins in voller Länge; Aktivität von β-Sekretasen.

3. Peptid zur Verwendung gemäß den Ansprüchen 1-2, wobei die Behandlung auch die durch β-Amyloidpeptide induzierte Produktion von Sauerstoffradikalen und die synaptische Dysfunktion hemmt.

4. Peptid zur Verwendung gemäß den Ansprüchen 1-3, wobei die Tau-Protein-abhängigen Erkrankungen eine oder mehrere sind aus: Altersbedingte Tau-Astrogliopathie, Alzheimer-Erkrankung, argyrophile Körnerkrankheit, chronischtraumatische Enzephalopathie, corticobasale Degeneration, diffuse Neurofilament-Verwicklungen mit Verkalkung, frontotemporale Demenz, globuläre gliale Tauopathien, mit Chromosom 17 verbundener Parkinsonismus, Pick-Erkrankung, postenzephalitischer Parkinsonismus, primär altersbedingte Tauopathie, progressive supranukleäre Lähmung.

5. Peptid zur Verwendung gemäß Anspruch 1-4, wobei das Peptid in einer Einheitsdosis zwischen 1 und 7.000 mg verabreicht wird.

6. Peptid zur Verwendung gemäß den Ansprüchen 1-5, wobei das Peptid in einer Tagesdosis von 0,1 bis 100 mg/kg verabreicht wird.

7. Peptid zur Verwendung gemäß den Ansprüchen 1-6, wobei das Peptid als Lösung oder Spray oder Aerosol zur intranasalen Verabreichung formuliert ist.

8. Peptid zur Verwendung gemäß den Ansprüchen 1-7, wobei sich das intranasal verabreichte Peptid in Hirnarealen einschließlich Kortex, Hippocampus, Caudate-Putamen, Kleinhirn ansammelt.

9. Peptid zur Verwendung gemäß den Ansprüchen 1-8, wobei die Behandlung keine täglichen Verabreichungen erfordert und bis zu 48 Stunden nach der Verabreichung wirksam ist.

10. Pharmazeutische Zusammensetzung, die das Aβ1-6_{A2V}(D) Peptid in einer für die intranasale Verabreichung geeigneten Form umfasst.

11. Pharmazeutische Zusammensetzung gemäß Anspruch 10, in Form einer intranasal instillierbaren Lösung, eines intranasalen Sprays, eines intranasalen Pulvers oder eines intranasalen Aerosols.

12. Pharmazeutische Zusammensetzung gemäß den Ansprüchen 10-11, wobei das Aβ1-6_{A2v}(D) Peptid in einer Konzentration von 10 bis 100 mg/ml vorliegt.

13. Pharmazeutische Zusammensetzung gemäß den Ansprüchen 10-12 in Form einer Einheitsdosis, die 1 bis 7.000 mg Aβ1-6_{A2V}(D) Peptid enthält.

## Revendications

1. Peptide Aβ1-6_{A2v}(D) pour une utilisation dans une méthode de traitement multicible des maladies liées à la protéine tau, ledit peptide étant administré par voie intranasale.

2. Peptide pour l'utilisation selon la revendication 1, la méthode multicible étant efficace pour inhiber chacun des éléments suivants : liaison des oligomères Aβ1-42 à la protéine tau, polymérisation de la protéine tau pleine longueur ; activité β-sécrétase.

3. Peptide pour l'utilisation selon les revendications 1-2, le traitement inhibant également la production de radicaux d'oxygène induite par les peptides β-amyloïdes et le dysfonctionnement synaptique.

4. Peptide pour l'utilisation selon les revendications 1-3, lesdites maladies liées à la protéine tau étant l'une ou plusieurs parmi : l'astrogliopathie tau liée au vieillissement, la maladie d'Alzheimer, la démence à grains argyrophiles, l'encéphalopathie traumatique chronique, la dégénérescence corticobasale, les enchevêtrements diffus de neurofilaments avec calcification, la démence frontotemporale, les tauopathies gliales globulaires, la maladie de Parkinson liée au chromosome 17, la maladie de Pick, la maladie de Parkinson post-encéphalitique, la tauopathie primaire liée au vieillissement, la paralysie supranucléaire progressive.

5. Peptide pour l'utilisation selon les revendications 1-4, ledit peptide étant administré sous forme d'une dose unitaire comprise entre 1 et 7 000 mg.

6. Peptide pour l'utilisation selon les revendications 1-5, ledit peptide étant administré à une dose quotidienne comprise entre 0,1 et 100 mg/kg.

7. Peptide pour l'utilisation selon les revendications 1-6, ledit peptide étant formulé sous forme d'une solution ou d'un spray ou d'un aérosol, pour une administration intranasale.

8. Peptide pour l'utilisation selon les revendications 1-7, ledit peptide administré par voie intranasale s'accumulant dans les zones du cerveau incluant le cortex, l'hippocampe, le striatum, le cervelet.

9. Peptide pour l'utilisation selon les revendications 1-8, ledit traitement ne nécessitant pas d'administrations quotidiennes, étant efficace jusqu'à 48 heures après l'administration.

10. Composition pharmaceutique comprenant le peptide Aβ1-6_{A2v}(D) sous une forme adaptée pour une administration intranasale.

11. Composition pharmaceutique selon la revendication 10, sous forme d'une solution pouvant être instillée par voir intranasale, d'un spray intranasal, d'une poudre intranasale, d'un aérosol intranasal.

12. Composition pharmaceutique selon les revendications 10-11, dans laquelle ledit peptide Aβ1-6_{A2v}(D) est présent à une concentration comprise entre 10 et 100 mg/ml.

13. Composition pharmaceutique selon les revendications 10-12, sous la forme d'une dose unitaire contenant de 1 à 7 000 mg de peptide Aβ1-6_{A2v}(D).
